# EUROPEAN PATENT APPLICATION

(11) **EP 4 113 532 A1**
(43) Date of publication of application: **04.01.2023**
(21) Application number: 21912467.4
(22) Date of filing: 20.01.2021
(51) Int. Cl.: G16H 40/40, A61B 18/12

(54) **METHOD AND DEVICE FOR DYNAMICALLY ADJUSTING RADIO FREQUENCY PARAMETERS, AND RADIO FREQUENCY HOST**

(30) Priority: 31.12.2020 CN 202011640959
(71) Applicant: Hangzhou Broncus Medical Co., Ltd., Hangzhou, Zhejiang 310051 (CN)
(72) Inventor: XU, Hong, Hangzhou, Zhejiang 310051 (CN); CUI, Changjie, Hangzhou, Zhejiang 310051 (CN)
(74) Representative: Wallinger Ricker Schlotter Tostmann
(86) International application number: PCT/CN2021/072954
(87) International publication number: WO 2022/141687

(57) **Abstract**

A method and an apparatus for dynamically adjusting a radio frequency parameter, and a radio frequency host are provided. The method for dynamically adjusting the radio frequency parameter includes the following steps: determining an operation stage of a radio frequency operation and acquiring a radio frequency data standard range and a radio frequency data limit range corresponding to an operation object of the radio frequency operation at the operation stage; detecting radio frequency data of the operation object in real time; controlling the radio frequency data to be within the radio frequency data standard range by controlling an injection volume of a syringe pump to the operation object when the radio frequency data detected in real time exceeds the radio frequency data standard range but does not exceed the radio frequency data limit range and lasts for a preset duration; and stopping outputting radio frequency energy when the radio frequency data detected in real time exceeds the radio frequency data limit range. Accordingly, the radio frequency data is dynamically adjusted within the radio frequency data standard range, and the effect of the radio frequency operation is improved.

## Description

### TECHNICAL FIELD

Embodiments of the present application relate to the field of electronic technology, and particularly to a method and an apparatus for dynamically adjusting a radio frequency parameter and a radio frequency host.

### BACKGROUND

Abnormalities may occur due to uncontrollability of an operation object during the radio frequency operation, which may cause the damage to a radio frequency host or the operation object, and even cause the damage to a radio frequency operator.

In the prior art, an alarm will be given when the abnormalities occurring during the radio frequency operation are detected. If the operator is unaware of the alarm for some reason, an operation risk to the operation object or the damage to the radio frequency host will be caused. Such a solution is insufficient for the protection against the abnormalities occurring during the radio frequency operation. As a result, the safety of the radio frequency operation cannot be guaranteed.

### SUMMARY

### Technical problem

Embodiments of the present application provide a method and an apparatus for dynamically adjusting a radio frequency parameter and a radio frequency host, which can achieve dynamically adjusting radio frequency data of a radio frequency object by comparing the detected radio frequency data of an operation object with a preset radio frequency data standard range and a preset radio frequency data limit range, so as to improve the success rate and the safety of the radio frequency operation.

### Technical solution

In one aspect, embodiments of the present application provide a method for dynamically adjusting a radio frequency parameter, including steps of:
Determining an operation stage of a radio frequency operation and acquiring a radio frequency data standard range and a radio frequency data limit range corresponding to an operation object of the radio frequency operation at the operation stage, wherein the radio frequency data standard range is within the radio frequency data limit range; detecting radio frequency data of the operation object in real time, and comparing the radio frequency data of the operation object with the radio frequency data standard range and the radio frequency data limit range; controlling the radio frequency data to be within the radio frequency data standard range by controlling an injection volume of a syringe pump to the operation object when the radio frequency data detected in real time exceeds the radio frequency data standard range but does not exceed the radio frequency data limit range and lasts for a preset duration; and stopping outputting radio frequency energy when the radio frequency data detected in real time exceeds the radio frequency data limit range.

In one aspect, embodiments of the present application further provide an apparatus for dynamically adjusting a radio frequency parameter, including:
an acquisition module, configured to determine an operation stage of a radio frequency operation and acquire a radio frequency data standard range and a radio frequency data limit range corresponding to an operation object of the radio frequency operation at the operation stage, wherein the radio frequency data standard range is within the radio frequency data limit range; a detection module, configured to detect radio frequency data of the operation object in real time; a comparison module, configured to compare the detected radio frequency data with the radio frequency data standard range and the radio frequency data limit range; and a control module, configured to control the radio frequency data to be within the radio frequency data standard range by controlling an injection volume of a syringe pump to the operation object when the radio frequency data detected in real time exceeds the radio frequency data standard range but does not exceed the radio frequency data limit range and lasts for a preset duration, and stop outputting radio frequency energy when the radio frequency data detected in real time exceeds the radio frequency data limit range.

In one aspect, embodiments of the present application further provide a radio frequency host, including:
a memory and a processor, wherein the memory stores an executable program code; and the processor coupled with the memory calls the executable program code stored in the memory to execute the method for dynamically adjusting the radio frequency parameter as described above.

### Beneficial effects

As can be known from the above embodiments of the present application that the radio frequency data standard range corresponding to the operation object of the radio frequency operation at the current operation stage is acquired, and the radio frequency data detected in real time is compared with the radio frequency data standard range and the radio frequency data limit range in real time, respectively. When the radio frequency data detected in real time exceeds the radio frequency data standard range but does not exceed the radio frequency data limit range and lasts for the preset duration, the radio frequency data is controlled to be within the radio frequency data standard range by controlling the injection volume of the syringe pump to the operation object. Accordingly, the radio frequency data is dynamically adjusted to be within the radio frequency data standard range, and the success rate of the radio frequency operation is improved. If the radio frequency data detected in real time exceeds the radio frequency data limit range, it is determined that abnormalities occur in the radio frequency host of the current radio frequency operation or the operation object, and the output of the radio frequency energy is stopped. Therefore, the radio frequency host and the operation object are prevented from being damaged, and the safety of the radio frequency operation is improved.

### BRIEF DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a schematic diagram illustrating an application scenario of a method for dynamically adjusting a radio frequency parameter according to an embodiment of the present application;
FIG. 2 is a schematic flow chart of a method for dynamically adjusting a radio frequency parameter according to an embodiment of the present application;
FIG. 3 is a schematic flow chart of a method for dynamically adjusting a radio frequency parameter according to another embodiment of the present application;
FIG. 4 is a structural schematic diagram of an apparatus for dynamically adjusting a radio frequency parameter according to an embodiment of the present application; and
FIG. 5 is a structural schematic diagram of a radio frequency host according to an embodiment of the present application.

### DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application. All other embodiments obtained by ordinary persons skilled in the art based on the embodiments of the present invention without creative efforts shall fall within the protection scope of the present invention.

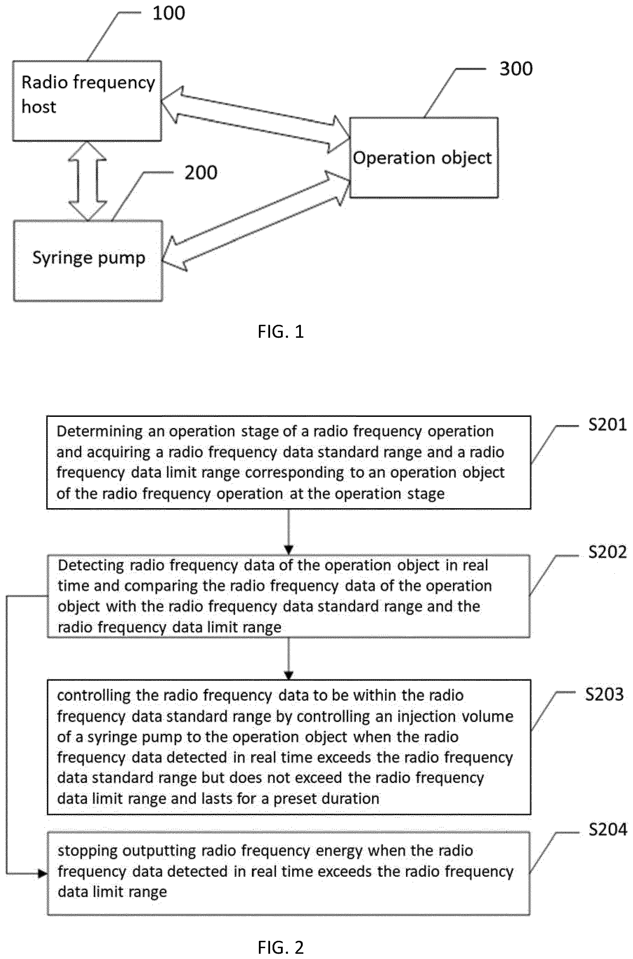

FIG. 1 is a schematic diagram illustrating an application scenario of a method for dynamically adjusting a radio frequency parameter according to an embodiment of the present application. The method for dynamically adjusting the radio frequency parameter may be configured to detect radio frequency data and compare the radio frequency data with a standard range and a limit range corresponding to a current operation stage during a radio frequency operation, and determine whether an abnormality occurs in the radio frequency operation, thereby interfering the radio frequency operation having the abnormality, such that the radio frequency operation may continue to be performed smoothly, the serious abnormality can be interrupted in time, and the safety of the radio frequency operation is improved.

Particularly, an execution main body of the method is a radio frequency host, and the radio frequency host may particularly be a device such as a radio frequency ablation instrument. As shown in FIG. 1, the radio frequency host 100 is connected with a syringe pump 200, and the radio frequency host 100 and the syringe pump 200 are also connected with the operation object 300. When the radio frequency operation starts, the radio frequency host 100 sends radio frequency energy to the operation object 300 by a radio frequency generation apparatus. The radio frequency host 100 controls the injection pump 200 to inject a cooling liquid to the operation object 300. The radio frequency host 100 has radio frequency data standard ranges and radio frequency data limit ranges for each of an initial stage, a middle stage and a final stage of the radio frequency operation on the operation object 300. During the radio frequency operation, when characters of the operation object 300 change, the radio frequency data acting on the operation object will change therewith.

Further, the radio frequency data standard range and the radio frequency data limit range each may be a value range, with a maximum value and a minimum value. If the real-time radio frequency data of the operation object 300 is greater than the maximum value or less than the minimum value of the standard range, it can enable the radio frequency data to be within the standard range by controlling an injection volume of the syringe pump. The injection volume may be controlled by controlling an injection flow rate. If the real-time radio frequency data of the operation object 300 is greater than the maximum value or less than the minimum value of the limit range, it is determined that an abnormality occurs in the radio frequency host 100 or the operation object 300, and the radio frequency operation should be stopped. The standard range and the limit range of the radio frequency data may further be radio frequency data change rate, i.e., slope. Within a preset detection duration, the real-time radio frequency data forms an excessive slope, which exceeds a preset slope, the purpose of adjusting the radio frequency data can also be achieved by adjusting the injection volume of the syringe pump, or the radio frequency operation is stopped to eliminate failures. Accordingly, the safety of the radio frequency operation is improved.

Reference is made to FIG. 2, which is a schematic flow chart of a method for dynamically adjusting a radio frequency parameter according to an embodiment of the present application. The method may be applied to a radio frequency host as shown in FIG. 1. As shown in FIG. 2, the method particularly includes the following steps.

In step S201, a current operation stage of a radio frequency operation is determined, and a radio frequency data standard range and a radio frequency data limit range corresponding to an operation object of the radio frequency operation and the operation stage are acquired.

The radio frequency data standard range is within the radio frequency data limit range, that is, the minimum value of the radio frequency data standard range is greater than the minimum value of the radio frequency data limit range, and the maximum value of the radio frequency data standard range is greater than the maximum value of the radio frequency data limit range.

Particularly, for different types of operation objects or individual differences of the same type of operation objects, the radio frequency data standard range will be different. For different radio frequency operation stages of the same operation object, it has different radio frequency data standard ranges and different radio frequency data limit ranges. The operation object may be any object subject to the radio frequency operation. For example, during radio frequency ablation, the operation object may be an abnormal tissue of a biological body, and the abnormal tissue is eliminated or reduced by means of ablation.

The radio frequency host has information on the radio frequency data standard ranges and the radio frequency data limit ranges of a specific operation object at different operation stages inside, for being read by a detection apparatus of the radio frequency host. The radio frequency data of the operation object of the current radio frequency operation and the operation stage is detected in real time, and is compared with the radio frequency data standard range and the radio frequency data limit range, respectively.

In step S202, the radio frequency data of the operation object is detected in real time, and compared with the radio frequency data standard range and the radio frequency data limit range, respectively.

The radio frequency operation will generate the radio frequency data when acting on the operation object, wherein the radio frequency data may particularly include an impedance value, a temperature value, a current value and a voltage value. The radio frequency host detects the above radio frequency data of the operation object in real time, and these radio frequency data indicates whether the current radio frequency operation is normal.

In step S203, if the radio frequency data detected in real time exceeds the radio frequency data standard range but does not exceed the radio frequency data limit range and lasts for a preset duration, the radio frequency data is controlled to be within the radio frequency data standard range by controlling the injection volume of the injection pump to the operation object.

When it is detected that the radio frequency data of the test object at the current operation stage exceeds the radio frequency data standard range and lasts for the preset duration, the radio frequency data is adjusted to return to the normal standard range by controlling the injection volume of the syringe pump, the instability of the radio frequency data due to accidental factors is further eliminated and the intelligence of detection is improved.

In step S204, if the radio frequency data detected in real time exceeds the radio frequency data limit range, stopping outputting the radio frequency energy.

If the radio frequency data detected in real time exceeds the radio frequency data standard range, it indicating that a serious abnormality occurs in the radio frequency operation, in order to protect the safety of the radio frequency host and the operation object, outputting the radio frequency energy is immediately stopped. Particularly, a detection module may send the radio frequency data to a processor of the radio frequency host, and the processor sends a stop signal to a radio frequency signal generation apparatus of the radio frequency host, such that the radio frequency signal generation apparatus stops outputting the radio frequency signal.

In the embodiment of the present application, the radio frequency data standard range corresponding to the operation object of the radio frequency operation at the current operation stage is acquired, and the radio frequency data detected in real time is compared with the radio frequency data standard range and the radio frequency data limit range, respectively. If the radio frequency detected in real time exceeds the radio frequency data standard range but does not exceed the radio frequency data limit range and lasts for the preset duration, the radio frequency data is controlled to fall within the radio frequency data standard range by controlling the injection volume of the syringe pump to the operation object. Accordingly, the radio frequency data is dynamically adjusted to fall within the radio frequency data standard range, and the success rate of the radio frequency operation is improved. If the radio frequency data detected in real time exceeds the radio frequency data limit range, it is determined that an abnormality exists in the radio frequency host of the current radio frequency operation or the operation object, and the output of the radio frequency energy is stopped. Therefore, the radio frequency host and the operation object are prevented from being damaged, and the safety of the radio frequency operation is improved.

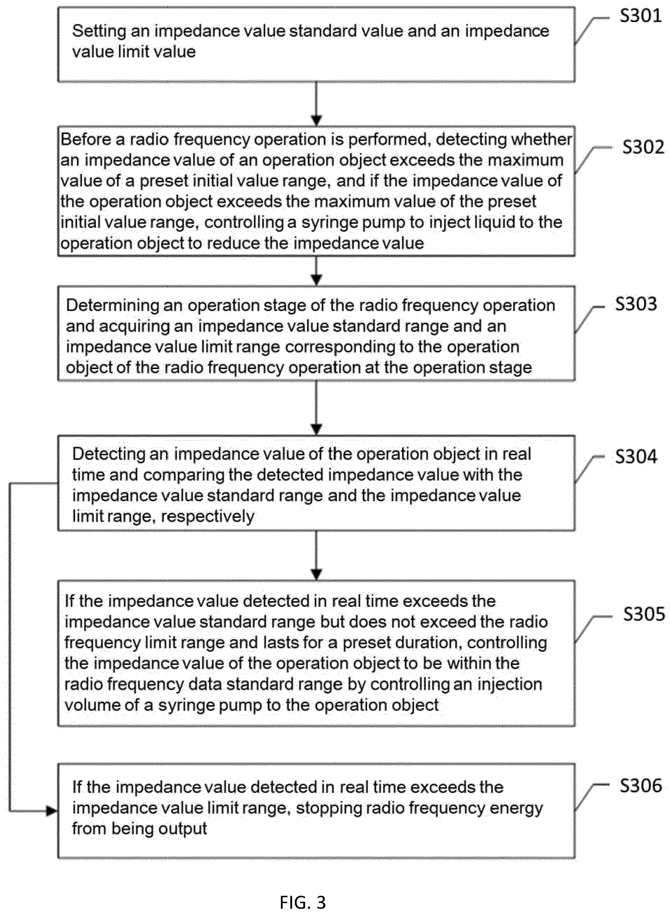

Reference is made to FIG. 3, which is a flow chart of an implementation of a method for dynamically adjusting a radio frequency parameter according to another embodiment of the present application. The method may be applied to a radio frequency host shown in FIG. 1. As shown in FIG. 3, the method particularly includes the following steps.

In step S301, an impedance value standard range and an impedance value limit range are set.

In response to a setting operation of a user, an input interface of the minimum value, the maximum value and a change rate of the impedance value are displayed, wherein the setting operation may be manually input by a user, or may be called from a memory of the radio frequency host or a database of a server connected with the radio frequency host according to an instruction from the user.

A first minimum value, a first maximum value and a first change rate of the impedance value set by the user are acquired, the first minimum value and the first maximum value input by the user are used as the minimum value and the maximum value of a standard range, and the first change rate input by the user is used as the standard slope.

A second minimum value, a second maximum value and a second change rate of the impedance value set by the user are acquired, the second minimum value and the second maximum value input by the user are respectively used as the minimum value and the maximum value of a limit range, and the second change rate set by the user is used as the standard slope.

Among them, the first minimum value is greater than the second minimum value, the first maximum value is less than the second minimum value, the first change rate is less than the second change rate, and the low change rate indicates that the value changes less per unit time.

Particularly, the impedance value is set corresponding to a model of the radio frequency host, a task of the radio frequency operation and a nature of the operation object, and an operation position of the radio frequency operation on the operation object. Such a correspondence relationship is known and may be manually input by the user or stored in related devices such as the radio frequency host or the server in advance.

In step S302, before the radio frequency operation is performed, it is detected whether the impedance value of the operation object exceeds the maximum value of a preset initial value range, and if yes, the syringe pump is controlled to inject a liquid to the operation object to reduce the impedance value.

If the detected impedance value of the operation object is greater than the maximum value of the preset initial value range, the syringe pump is controlled to inject liquid to the operation object to reduce the impedance value of the operation object, until the impedance value returns to the preset initial value range. That is, before the radio frequency operation starts, the initial impedance value of the operation object is set to fall within the normal initial value range, so as to reduce the influence on the detection and the determination based on the impedance value during the radio frequency operation due to the deviation of the initial impedance value from the normal range after the radio frequency operation starts.

The initial value range corresponds to the nature of the operation object and a specific position of the radio frequency operation on the operation object, and is a common range obtained based on actual measurement values of a plurality of operation objects. For example, when the radio frequency operation is radio frequency ablation, the operation object is a human body and a specific location is a lung tissue, and the initial value range is 250Ω to 350Ω (ohm).

In step S303, a current operation stage of the radio frequency operation is determined, and an impedance value standard range and an impedance value limit range corresponding to the operation object of the radio frequency operation at the operation stage are acquired.

The impedance value standard range may be a standard value range of the impedance value, which includes a minimum value and a maximum value. During the radio frequency operation, the impedance value standard range is preferably 150Ω to 500Q. Alternatively, a standard slope set according to the nature of the operation object, that is, a standard change rate of the impedance value without being adjusted is determined according to the nature of the operation object under a premise that the impedance value of the operation object is not less than 150Ω and is not greater than 500Ω during the radio frequency operation. It is not required to make adjustment on a real-time change rate of the impedance value below the standard change rate for the operation object. The standard change rate is the standard slope.

The impedance value limit range may be a limit value range of the impedance value, which includes a minimum value and a maximum value. During the radio frequency operation, the impedance value limit range is preferably 50Q to 600Q. Alternatively, a limit slope set according to the nature of the operation object, that is, a limit change rate of a safe impedance value is determined according to the nature of the operation object under a premise that the impedance value of the operation object is not less than 50Ωand is not greater than 600Ω during the radio frequency operation. A real-time change rate of the impedance value below the standard change rate is safe for the operation object. The standard change rate is the standard slope.

Specific values of standard value range, the standard slope, the limit value range and the limit slope of the impedance value are related to the operation object and the operation stage at which the radio frequency operation is performed, and will be not particularly limited.

In step S304, the impedance value of the operation object is detected in real time, and the detected impedance value is compared with the impedance value standard range and the impedance value limit range, respectively.

The impedance value of the operation object may be directly detected by an impedance detection circuit, or a current value of the operation object may be detected by a current detection circuit, a voltage value of the operation object may be detected by a voltage detection circuit, and the impedance value of the operation object is calculated according to a formula representing the relationship of the current value, the voltage value and the impedance value.

The impedance value detected in real time is compared with the standard value range of the impedance value corresponding to the current operation stage of the operation object and/or the standard slope of the impedance value in real time, and compared with the limit value range of the impedance value corresponding to the current operation stage of the operation object and/or the limit slope of the impedance value.

In step S305, if the impedance value detected in real time exceeds the impedance value standard range but does not exceed the radio frequency data limit range and lasts for the preset duration, the impedance value of the operation object is controlled to return to the radio frequency data standard range by controlling the injection volume of the injection pump to the operation object.

Particularly, if the impedance value of the operation object detected in real time is less than the minimum value of the standard value range and/or a decrease rate of the impedance value of the operation object is greater than the first standard slope and lasts for the preset duration, the injection pump is controlled to reduce the amount of the liquid injected to the operation object according to a preset first injection volume.

Either one or both of two cases that the impedance value of the operation object is less than the minimum value of the standard value range and lasts for the preset duration and that the decrease rate of the impedance value of the operation object is greater than the first standard slope and lasts for the preset duration indicates or indicate that the impedance value of the operation object is too small or decreases too quickly and it is needed to increase the impedance value to reduce the injection volume of the injection pump to the operation object. The injection volume may be controlled by controlling an injection flow rate of the liquid. Within a fixed duration, the greater the flow rate is, the greater the injection volume is.

If the impedance value of the operation object detected in real time is greater than the maximum value of the standard value range and/or the increase rate of the impedance value of the operation object is greater than the second standard slope and lasts for the preset duration, the injection pump is controlled to increase the amount of the liquid injected to the operation object according to a preset second injection volume.

Either one or both of two cases that the impedance value of the operation object is greater than the maximum value of the standard value range and lasts for the preset duration and the increase rate of the impedance value of the operation object is greater than the second standard slope and lasts for the preset duration indicates or indicate that the impedance value of the operation object is too large or increases too rapidly, and it is needed to reduce the impedance value to increase the injection volume of the injection pump to the operation object.

Further, the impedance value may change due to accidental factors. In order to prevent the instability of a working process of the syringe pump due to frequent adjustment on the impedance value from influencing the effect of the radio frequency operation, the step of dynamically adjusting the impedance value of the operation object is started after the accidental factors is eliminated after the preset duration is ended.

If the impedance value of the operation object is not be controlled to fall within the radio frequency data standard range by controlling the injection volume of the injection pump to the operation object after a preset adjustment duration is ended, the radio frequency operation is stopped.

In step S306, if the impedance value detected in real time exceeds the impedance value limit range, output of the radio frequency energy is stopped.

Particularly, the limit slopes of the impedance value includes a first limit slope used to indicate a decrease rate of the impedance value and a second limit slope used to indicate the increase rate of the impedance value. The limit value range, the first limit slope and the second limit slope are limit abnormal values in nature, that is, no matter what operation stage at which the impedance value of the operation object detected in real time is, the output of the radio frequency energy to the operation object has to be stopped immediately provided that at least one of the following conditions is met. A first one of the conditions may include: the impedance value is greater than the maximum value of the limit value range, the impedance value is less than the minimum value of the limit value range, the decrease rate of the impedance value is greater than the first limit slope, and the increase rate of the impedance value is greater than the second limit slope. The decrease rate of the impedance value is a ratio of a decrease of the impedance value of the operation object within a duration unit to the unit duration; and the increase rate of the impedance value is a ratio of an increase of the impedance value of the operation object within a duration unit to the unit duration.

Further, while stopping outputting the radio frequency energy to the operation object, a text prompt is displayed and an audible and visual alarm is provided. Particularly, when the impedance value of the operation object detected in real time is less than the minimum value of the limit value range or the decrease rate of the impedance value of the operation object detected in real time is greater than the first limit slope, a first text prompt is displayed and the audible and visual alarm is provided.

When the impedance value of the operation object detected in real time is greater than the maximum value of the limit value range or the increase rate of the impedance value of the operation object detected in real time is greater than the first limit slope, a second text prompt is displayed and the audible and visual alarm is provided.

The first text prompt for example displays a text "LLL" on a display screen of the radio frequency host, and the second text prompt for example displays a text "HHH" on the display screen of the radio frequency host. The audible and visual alarm includes both an audible alarm and a visual alarm.

Further, when it is detected that the impedance value of the operation object exceeds the above non-limit abnormal value, the text prompt may be displayed and the audible and visual alarm may be given. Particularly, the contents and forms of the text prompt and the audible and visual alarm may be different from those when the impedance value of the operation object exceeds the limit abnormal value. The forms of the audible and visual alarm are distinguished too.

Further, after it is detected that the impedance value of the operation object exceeds the above-mentioned non-limit abnormal value and lasts for the preset duration, the output of the radio frequency energy to the operation object is stopped, and the text prompt is displayed and the audible and visual alarm is given. At this time, the contents and forms of the text prompt and the audible and visual alarm are the same as those when the impedance value of the operation object exceeds the limit abnormal value. The forms of the audible and visual alarm are also the same.

Further, if the detected impedance value of the operation object exceeds the radio frequency data standard range, a correspondence relationship between the impedance value of the operation object and the time of the radio frequency operation is displayed on a display interface in the form of a line with a first color; and if the detected impedance value of the operation object does not exceed the radio frequency data standard range, a correspondence relationship between the impedance value of the operation object and the time of the radio frequency operation is displayed on the display interface in the form of a line with a second color.

The reflectivity of the first color is greater than that of the second color, the greater the reflectivity is, the brighter the light is, and the higher a reminding degree to human eyes is. For example, a red color reflects 67% of light, a yellow color reflects 65% of light, a green color reflects 47% of light, and a cyan color reflects 36% of light. The first color may be red or yellow, and the second color may be green or cyan.

For technical details of the above steps, reference is made to the description of the embodiment as shown in FIG. 2, which will be omitted here.

In the embodiment of the present application, before the radio frequency operation is performed, the impedance value of the operation object is reduced to fall within the normal initial value range by means of injecting the liquid by the syringe pump, so as to improve the accuracy of detecting the impedance value during the subsequent radio frequency operation and improve the success rate of the radio frequency operation. The standard value range of the impedance value and the standard change slope of the impedance value corresponding to the operation object of the radio frequency operation at the current operation stage are acquired. The impedance value of the operation object detected in real time is compared with the standard value range and/or the standard slope, and with the limit value range and/or the limit slope in real time. The radio frequency data is controlled to fall within the radio frequency data standard range by controlling the injection volume of the syringe pump to the operation object. Accordingly, the radio frequency data is dynamically adjusted to be within the radio frequency data standard range, and the success rate of the radio frequency operation is improved. If the impedance value of the operation object detected in real time exceeds the limit value range and/or the change rate of the impedance value is greater than the limit slope, it is determined that an abnormality occurs in the radio frequency host of the current radio frequency operation or the operation object, the output of the radio frequency energy is stopped. As a result, the radio frequency host and the operation object are prevented from being damaged, and the safety of the radio frequency operation is improved. The text prompt is displayed and the audible and visual alarm is given, which further remind a radio frequency operator of paying attention to the safety of the radio frequency operation.

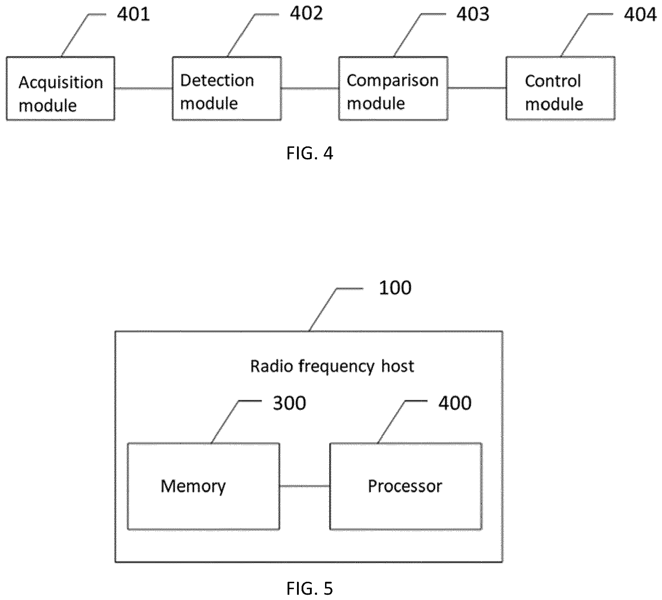

Reference is made to FIG. 4, which is a schematic diagram showing a structure of an apparatus for dynamically adjusting a radio frequency parameter according to an embodiment of the present application. For the convenience of the illustration, parts related to the embodiment of the present application are only shown. The apparatus may be provided in the above radio frequency host. The apparatus includes:
an acquisition module 401, which is configured to determine an operation stage at which a radio frequency operation is and acquire a radio frequency data standard range and a radio frequency data limit range corresponding to an operation object of the radio frequency operation at the operation stage, wherein the radio frequency data standard range is within the radio frequency data limit range;
a detection module 402, which is configured to detect radio frequency data of the operation object in real time;
a comparison module 403, which is configured to compare the detected radio frequency data with the radio frequency data standard range and the radio frequency data limit range; and
a control module 404, which is configured to control the radio frequency data to fall within the radio frequency data standard range by controlling an injection volume of the syringe pump to the operation object when the radio frequency data detected in real time exceeds the radio frequency data standard range but does not exceed the radio frequency data limit range and lasts for a preset duration, and stop output radio frequency energy when the radio frequency data detected in real time exceeds the radio frequency data limit range.

Further, the acquisition module 401 is further configured to acquire a standard value range of an impedance value of the operation object, a standard change slope of the impedance value of the operation object, a limit value range of the impedance value of the operation object, and a limit slope of the impedance value change of the operation object at the operation stage.

The acquisition module 401 is further configured to display an input interface of the minimum value, the maximum value and the change rate of the impedance value in response to a setting operation of a user, and acquire a first minimum value, a first maximum value, a first change rate, a second minimum value, a second maximum value and a second change rate, wherein the first minimum value and the first maximum value are taken as the minimum value and the maximum value of the standard value range and the first change rate input by the user is taken as the standard slope.

The second minimum value and the second maximum value are taken as the minimum value and the maximum value of the limit value range, and the second change rate input by the user is taken as the limit slope.

Further, the limit slope includes a first limit slope for indicating a decrease rate of the impedance value and a second limit slope for indicating an increase rate of the impedance value. The control module 404 is further configured to stop outputting radio frequency energy to the operation object when the impedance value of the operation object detected in real time meets at least one of preset conditions, wherein the preset conditions include:
the impedance value of the operation object detected in real time exceeds the limit value range, the decrease rate of the impedance value of the operation object detected in real time is greater than the first limit slope, and the increase rate of the impedance value of the operation object detected in real time is greater than the second limit slope.

Further, the apparatus further includes an early warning module (not shown in the drawing), wherein
the early warning module is configured to display a first text prompt and give an audible and visual alarm when the impedance value of the operation object detected in real time is less than the minimum value of the limit value range or the decrease rate of the impedance value of the operation object detected in real time is greater than the first limit slope; and
to display a second text prompt and give the audible and visual alarm when the impedance value of the operation object detected in real time is greater than the maximum value of the limit value range or the increase rate of the impedance value of the operation object detected in real time is greater than the second limit slope.

Further, the standard slope includes a first standard slope for indicating a decrease rate of the impedance value and a second standard slope for indicating an increase rate of the impedance value. The control module 404 is further configured to control the injection pump to reduce the amount of the liquid injected to the operation object according to a preset first injection volume when the impedance value of the operation object detected in real time is less than the minimum value of the standard value range and/or a decrease rate of the impedance value of the operation object is greater than the first standard slope and lasts for the preset duration; and
to control the injection pump to increase the amount of the liquid injected to the operation object according to a preset second injection volume when the impedance value of the operation object detected in real time is greater than the maximum value of the standard value range and/or an increase rate of the impedance value of the operation object is greater than the second standard slope and lasts for the preset duration.

Further, the detection module 402 is further configured to detect whether the impedance value of the operation object exceeds the maximum value of a preset initial value range before the radio frequency operation is performed.

The control module 404 is further configured to control the syringe pump to inject liquid to the operation object to reduce the impedance value when the impedance value of the operation object exceeds the maximum value of the preset initial value range, until the impedance value meets a preset initial value range.

Further, the apparatus further includes a display module (not shown in the drawing), wherein the display module is configured to display a correspondence relationship between the impedance value of the operation object and the time of the radio frequency operation on a display interface in the form of a line with a first color when the detected impedance value of the operation object exceeds the radio frequency data standard range;
and display a correspondence relationship between the impedance value of the operation object and the time of the radio frequency operation on the display interface in the form of a line with a second color when the detected impedance value of the operation object does not exceed the radio frequency data standard range; and
wherein the reflectivity of the first color is greater than that of the second color.

In the embodiments of the present application, before the radio frequency operation is performed, the impedance value of the operation object is reduced to fall within the normal initial value range by means of injecting liquid by the syringe pump, so as to improve the accuracy of detecting the impedance value during the subsequent radio frequency operation and improve the success rate of the radio frequency operation. The standard value range of the impedance value and the standard change slope of the impedance value corresponding to the operation object of the radio frequency operation at the current operation stage are acquired. The impedance value of the operation object detected in real time is compared with the standard value range and/or the standard slope and with the limit value range and/or the limit slope in real time. The radio frequency data is controlled to fall within the radio frequency data standard range by controlling the injection volume of the syringe pump to the operation object. Accordingly, the radio frequency data is dynamically adjusted to be within the radio frequency data standard range, and the success rate of the radio frequency operation is improved. If the impedance value of the operation object detected in real time exceeds the limit value range and/or the change rate of the impedance value is greater than the limit slope, it is determined that an abnormality occurs in the radio frequency host of the current radio frequency operation or the operation object, and the output of the radio frequency energy is stopped. As a result, the radio frequency host and the operation object are prevented from being damaged, and the safety of the radio frequency operation is improved. The text prompt is displayed and the audible and visual alarm is provided, which further remind a radio frequency operator of paying attention to the safety of the radio frequency operation.

As shown in FIG. 5, embodiments of the present application further provide a radio frequency host, including a memory 300 and a processor 400. The processor 400 may be a control module 404 of any apparatus for dynamically adjusting the radio frequency parameter provided in the above embodiments. The memory 300 may be for example a hard disk drive memory, a non-volatile memory (such as a flash memory or another electronically programmable and restricted delete memory used to form a solid-state drive and the like), a volatile memory (such as a static or dynamic random access memory and the like) and the like, which will not be limited in the embodiment of the present application.

The memory 300 stores an executable program code; and the processor 400 coupled with the memory 300 calls the executable program code stored in the memory to execute the method for dynamically adjusting the radio frequency parameter as described above.

Moreover, embodiments of the present application further provide a computer-readable storage medium. The computer-readable storage medium may be provided in the radio frequency host in each of the above embodiments, and the computer-readable storage medium may be a memory 300 in the embodiment as shown in FIG. 5. A computer program is stored in the computer-readable storage medium, and when being executed by the processor, implements the method for dynamically adjusting the radio frequency parameter according to the embodiments as shown in FIG. 2 and FIG. 3. Further, the computer-readable storable medium may further be a U disk, a mobile hard disk, a read-only memory (ROM), a RAM, a magnetic disk or an optical disk, and other various media that may store the program code.

It should be noted that for simplicity of description, the foregoing method embodiments are all expressed as a series of action combinations, however, according to the present application, some steps may be performed in other sequences or simultaneously, those skilled in the art should appreciate that the present application is not limited by the described sequence of actions. Secondly, those skilled in the art should further appreciate that the embodiments described in the specification are all preferred embodiments, and the involved actions and modules are not necessarily all required by the present application.

In the above-mentioned embodiments, descriptions of the embodiments have particular emphasis respectively. For parts that are not described in detail in a certain embodiment, reference may be made to related descriptions of other embodiments.

The above are descriptions of the method and the apparatus for dynamically adjusting the radio frequency parameter and the radio frequency host according to the present application. For those skilled in the art, changes may be made to specific implementations and application scopes according to the ideas of the embodiments of the present application. In summary, the content of this specification should not be construed as a limitation on the present application.

## Claims

1. A method for dynamically adjusting a radio frequency parameter, comprising steps of:
determining an operation stage of a radio frequency operation and acquiring a radio frequency data standard range and a radio frequency data limit range corresponding to an operation object of the radio frequency operation at the operation stage, wherein the radio frequency data standard range is within the radio frequency data limit range;
detecting radio frequency data of the operation object in real time, and comparing the radio frequency data of the operation object with the radio frequency data standard range and the radio frequency data limit range;
controlling the radio frequency data to be within the radio frequency data standard range by controlling an injection volume of a syringe pump to the operation object when the radio frequency data detected in real time exceeds the radio frequency data standard range but does not exceed the radio frequency data limit range and lasts for a preset duration; and
stopping outputting radio frequency energy when the radio frequency data detected in real time exceeds the radio frequency data limit range.

2. The method according to claim 1, wherein the radio frequency data of the operation object comprises an impedance value of the operation object, and the step of acquiring the radio frequency data standard range and the radio frequency data limit range corresponding to the operation object of the radio frequency operation at the operation stage comprises:
acquiring a standard value range of the impedance value of the operation object and a standard change slope of the impedance value of the operation object at the operation stage; and
acquiring a limit value range of the impedance value of the operation object and a limit change slope of the impedance value of the operation object at the operation stage.

3. The method according to claim 2, comprising, before the step of determining the operation stage of the radio frequency operation, steps of:
displaying an input interface of a minimum value, a maximum value and a change rate of the impedance value in response to a setting operation of a user, and acquiring a first minimum value, a first maximum value, a first change rate, a second minimum value, a second maximum value and a second change rate; and
taking the first minimum value and the first maximum value as the minimum value and the maximum value of the standard value range respectively, and taking the first change rate input by the user as the standard slope; and
taking the second minimum value and the second maximum value as the minimum value and the maximum value of the limit value range respectively, and taking the second change rate input by the user as the limit slope.

4. The method according to claim 3, wherein the limit slope comprises a first limit slope for indicating a decrease rate of the impedance value, and a second limit slope for indicating an increase rate of the impedance value, and wherein the step of stopping outputting the radio frequency energy comprises:
stopping outputting radio frequency energy to the operation object when the impedance value of the operation object detected in real time meets at least one of preset conditions comprising: the impedance value of the operation object detected in real time exceeds the limit value range, the decrease rate of the impedance value of the operation object detected in real time is greater than the first limit slope, and the increase rate of the impedance value of the operation object detected in real time is greater than the second limit slope.

5. The method according to claim 4, further comprising steps of:
displaying a first text prompt and providing an audible and visual alarm when the impedance value of the operation object detected in real time is less than the minimum value of the limit value range or the decrease rate of the impedance value of the operation object detected in real time is greater than the first limit slope; and
displaying a second text prompt and providing an audible and visual alarm when the impedance value of the operation object detected in real time is greater than the maximum value of the limit value range or the increase rate of the impedance value of the operation object detected in real time is greater than the first limit slope.

6. The method according to claim 5, wherein the standard slope comprises a first standard slope for indicating a decrease rate of the impedance value, and a second standard slope for indicating an increase rate of the impedance, and wherein the step of controlling the radio frequency data to be within the radio frequency data standard range by controlling the injection volume of the syringe pump to the operation object when the radio frequency data detected in real time exceeds the radio frequency data standard range but does not exceed the radio frequency data limit range and lasts for a preset duration comprises:
controlling the injection pump to reduce an amount of the liquid injected to the operation object according to a preset first injection volume when the impedance value of the operation object detected in real time is less than the minimum value of the standard value range and/or a decrease rate of the impedance value of the operation object is greater than the first standard slope and lasts for the preset duration; and
controlling the injection pump to increase the amount of the liquid injected to the operation object according to a preset second injection volume when the impedance value of the operation object detected in real time is greater than the maximum value of the standard value range and/or an increase rate of the impedance value of the operation object is greater than the second standard slope and lasts for the preset duration.

7. The method according to claim 1, further comprising, before the radio frequency operation is performed, steps of:
detecting whether the impedance value of the operation object exceeds the maximum value of a preset initial value range; and
controlling the syringe pump to inject liquid to the operation object to reduce the impedance value when the impedance value of the operation object exceeds the maximum value of the preset initial value range, until the impedance value meets the preset initial value range.

8. The method according to claim 1, further comprising, after the detected radio frequency data is compared with the radio frequency data standard range in real time, steps of:
displaying a correspondence relationship between the impedance value of the operation object and the time of the radio frequency operation on a display interface in the form of a line with a first color when the detected impedance value of the operation object exceeds the radio frequency data standard range;
displaying a correspondence relationship between the impedance value of the operation object and the time of the radio frequency operation on the display interface in the form of a line with a second color when the detected impedance value of the operation object does not exceed the radio frequency data standard range; and
wherein a reflectivity of the first color is greater than a reflectivity of the second color.

9. An apparatus for dynamically adjusting a radio frequency parameter, comprising:
an acquisition module, configured to determine an operation stage of a radio frequency operation and acquire a radio frequency data standard range and a radio frequency data limit range corresponding to an operation object of the radio frequency operation at the operation stage, wherein the radio frequency data standard range is within the radio frequency data limit range;
a detection module, configured to detect radio frequency data of the operation object in real time;
a comparison module, configured to compare the detected radio frequency data with the radio frequency data standard range and the radio frequency data limit range; and
a control module, configured to control the radio frequency data to be within the radio frequency data standard range by controlling an injection volume of a syringe pump to the operation object when the radio frequency data detected in real time exceeds the radio frequency data standard range but does not exceed the radio frequency data limit range and lasts for a preset duration, and stop outputting radio frequency energy when the radio frequency data detected in real time exceeds the radio frequency data limit range.

10. A radio frequency host, comprising:
a memory and a processor, wherein
the memory stores an executable program code; and
the processor coupled with the memory calls the executable program code stored in the memory to execute the method for dynamically adjusting the radio frequency parameter according to any one of claims 1 to 8.
